# FASCICULE DE BREVET EUROPEEN

(11) **EP 2 528 501 B1**
(45) Date de publication et mention de la délivrance du brevet: **04.12.2013**
(21) Numéro de dépôt: 11708516.7
(22) Date de dépôt: 24.01.2011
(51) Int. Cl.: A61B 5/0478, A61F 7/12, A61N 1/05

(54) **ELECTRODE INTRACEREBRALE**
GEHIRNELEKTRODE
INTRACEREBRAL ELECTRODE

(30) Priorité: 25.01.2010 FR 1050450
(43) Date de publication de la demande: 05.12.2012
(73) Titulaire: Assistance Publique - Hôpitaux de Paris, 75184 Paris (FR); Dixi Microtechniques S.A.S., 25000 Besançon (FR)
(72) Inventeur: CARPENTIER, Alexandre, F-75116 Paris (FR); BOILLON, Christophe, F-25640 Pouligney (FR)
(74) Mandataire: Koelbel, Caroline
(86) Numéro de dépôt international: PCT/FR2011/000044
(87) Numéro de publication internationale: WO 2011/089342

(56) Documents cités:
- WO-A1-2007/061982
- US-A1- 2004 010 208
- US-A1- 2004 082 984
- US-A1- 2009 005 843
- US-B1- 6 682 508

## Description

### Domaine technique :

La présente invention concerne une électrode intracérébrale comprenant un corps étroit et allongé destiné à être implanté dans le cerveau d'un patient, ce corps étant pourvu de plots de contact reliés électriquement à un appareil de mesure, et un organe de montage agencé pour fixer ledit corps au crâne du patient, ledit corps de l'électrode étant creux pour recevoir un instrument de traitement et étant pourvu d'une extrémité distale fermée.

### Technique antérieure :

Ces électrodes intracérébrales sont connectées à un appareil de mesure du type électroencéphalogramme et sont largement utilisées pour détecter des zones à traiter dans le cerveau, notamment des zones de dysfonctionnement à l'origine de symptômes neurologiques ou psychiatriques. Ces électrodes sont de plus en plus utilisées en combinaison avec d'autres instruments de traitement pour détecter la zone à traiter et effectuer le traitement presque simultanément, sans avoir besoin de retirer l'électrode, ce qui permet de cibler avec une grande précision la zone traitée. Un exemple est illustré dans la publication US 2004/0215162 qui permet d'envoyer un fluide de traitement ou de retirer une partie du liquide cérébral par un cathéter introduit dans une électrode intracérébrale creuse et dont l'extrémité distale est ouverte pour le passage du cathéter. Un exemple similaire est illustré dans la publication US 5,843,150. Un autre exemple, décrit par la publication US 2004/0082984, concerne une électrode intracérébrale permettant de traiter certaines zones du cerveau au moyen d'un fluide réfrigéré de manière appropriée. Ce fluide circule en boucle entre un réservoir externe et l'électrode à l'intérieur de laquelle il chemine en traversant d'abord un tube central puis un canal périphérique avant de retourner dans ledit réservoir. Une telle électrode ne permet cependant pas de traiter conjointement une zone lésée au moyen d'un instrument de traitement supplémentaire introduit à l'intérieur de l'électrode. Par ailleurs, le document WO 2007/061982 enseigne la possibilité de réaliser une ablation d'une zone du cerveau de manière concomitante à un enregistrement de l'activité cérébrale, au moyen d'une électrode implantée dans le cerveau à travers le système veineux. Là encore, aucun système de protection des zones environnantes à la zone traitée n'est prévu.

Un autre exemple est illustré dans la publication WO 2007/020363 qui permet de pratiquer une thermolésion par un faisceau laser ou par cryogénisation, l'instrument de traitement (fibre optique ou similaire) étant monté dans une électrode intracérébrale creuse pourvue d'une fenêtre latérale transparente aux rayonnements. Ce type de traitement est par exemple utilisé pour traiter un foyer épileptogène au niveau de l'hippocampe. Toutefois, l'application de la source de chaleur ou de froid doit être très précisément ciblée, contrôlée et maîtrisée, pour ne pas détruire les zones cérébrales environnantes par nécrose, pour respecter les zones cérébrales saines ainsi que les artères intracraniennes. En particulier, le dégagement de chaleur ou de froid produit par l'instrument de traitement risque par conduction au travers de l'électrode intracérébrale d'abîmer de manière irréversible les tissus périphériques à la zone volontairement traitée. Un dégagement de chaleur ou de froid trop important risque aussi d'altérer le fonctionnement de l'électrode intracérébrale pouvant aller jusqu'à sa destruction. Une attention particulière devra être portée lors de l'arrêt du traitement, du fait que les plots de contact dissipent très lentement l'énergie thermique accumulée. Ces plots de contact devront avoir des caractéristiques particulières, permettant d'obtenir la meilleure image possible au contrôle par un système d'imagerie à résonance magnétique (IRM).

### Exposé de l'invention :

La présente invention vise à résoudre ce problème en proposant d'associer à une électrode intracérébrale spécifique des moyens de régulation thermique pour supprimer tout risque d'irradiation par quelle source que ce soit des zones du cerveau disposées en périphérie de la zone traitée.

Dans ce but, l'invention concerne une électrode intracérébrale selon la revendication 1. Ladite électrode comporte un circuit intérieur de circulation de fluide fermé ménagé à l'intérieur dudit corps de l'électrode jusqu'à proximité de ladite extrémité distale fermée, et des moyens de connexion extérieurs audit corps pour connecter ledit circuit intérieur de fluide fermé à un circuit extérieur de circulation de fluide de sorte que ledit fluide, amené à travers ledit circuit extérieur de circulation de fluide, circule à l'intérieur de ladite électrode à travers ledit circuit intérieur de circulation de fluide.

Grâce à cette circulation intérieure de fluide, il est possible de contrôler et de maîtriser la dissipation de chaleur ou de froid par l'électrode et d'empêcher tout dégât collatéral dû à un traitement à l'aide d'un instrument, tel que par exemple une fibre optique, introduit dans le corps creux de l'électrode. En effet, cette circulation de fluide permet de limiter la capacitance thermique du tissu traité en le refroidissant pendant et immédiatement après le traitement pour éviter une diffusion thermique aux tissus sains avoisinants. De plus, le refroidissement permet d'éviter tout phénomène d'ébullition, c'est-à-dire de création de bulle d'air (effet pop-corn) qui constituent un risque de saignement induit et un risque d'artefact sur les images d'IRM. Enfin, ce refroidissement permet d'éviter tout phénomène de carbonisation du tissu traité au contact de la sonde et par là même d'éviter la création d'une adhérence de la sonde au tissu traité.

Ledit circuit intérieur de circulation de fluide comporte un tube creux délimitant un canal central pourvu au moins d'un orifice d'entrée et d'un orifice de sortie, la paroi de ce tube est une paroi double délimitant au moins un canal périphérique pourvu au moins d'un orifice d'entrée et d'un orifice de sortie, lesdits moyens de connexion comportent l'orifice d'entrée d'un des canaux et l'orifice de sortie de l'autre canal, et lesdits canaux communiquent entre eux par l'orifice de sortie d'un des canaux et l'orifice d'entrée de l'autre canal.

Dans une première variante de réalisation, ledit tube forme le corps de ladite électrode, alors que dans une seconde variante de réalisation, il forme une pièce distincte logée dans le corps creux de ladite électrode.

Le tube peut comporter au moins deux canaux périphériques, un des canaux faisant partie des moyens de circulation du fluide, l'autre canal délimitant un passage étanche et isolé pour les fils d'alimentation électrique des plots de contact.

De manière avantageuse, ledit corps de l'électrode et/ou ledit tube sont réalisés dans des matériaux perméables aux rayonnements magnétiques. Ils peuvent également être réalisés dans des matériaux perméables aux rayonnements laser.

L'électrode comporte, de préférence, un bouchon agencé pour fermer ledit corps et/ou ledit tube de manière étanche, sous vide d'air ou non.

L'électrode peut comporter au moins une fenêtre latérale à proximité de son extrémité distale transparente aux rayonnements laser, et lesdits plots de contact peuvent être collés ou surmoulés sur ledit corps de l'électrode. Dans une autre variante de réalisation, les plots de contact et/ou les fils électriques peuvent être formés par dépôt de métal sous vide.

### Description sommaire des dessins :

La présente invention et ses avantages apparaîtront mieux dans la description suivante de deux modes de réalisation donnés à titre d'exemple non limitatif, en référence aux dessins annexés, dans lesquels :
- la figure 1 est une vue en élévation d'une électrode intracérébrale selon l'invention en une pièce,
- la figure 2 est une vue en élévation d'une variante de réalisation de l'électrode intracérébrale selon l'invention en deux pièces,
- les figures 3A et 3B sont des vues en coupe axiale de l'électrode de la figure 1, respectivement de sa partie haute et de sa partie basse, et
- la figure 4 est une vue en coupe radiale de l'électrode de la figure 1.

### Illustrations de invention et différentes manières de la réaliser :

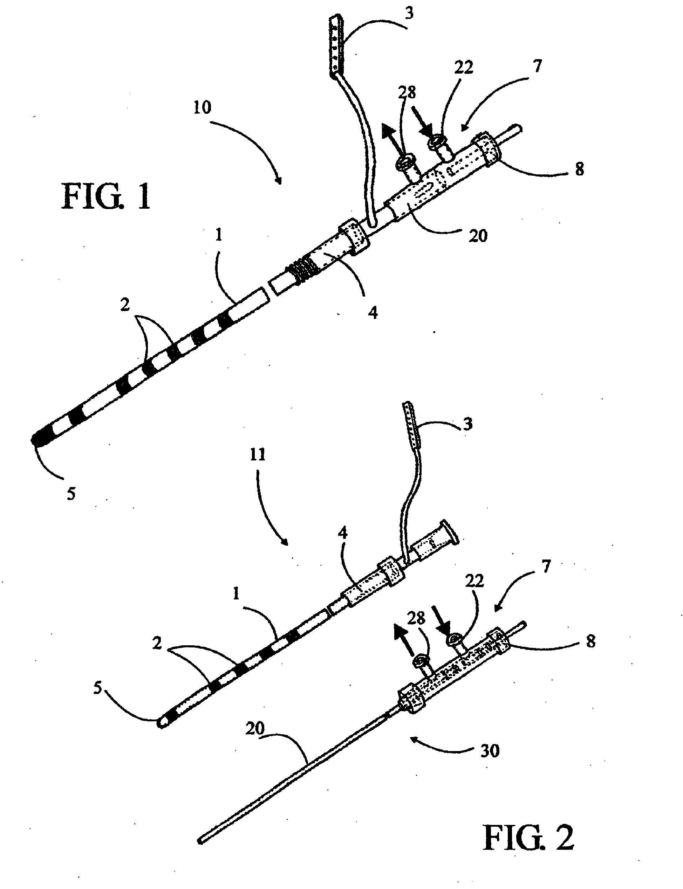

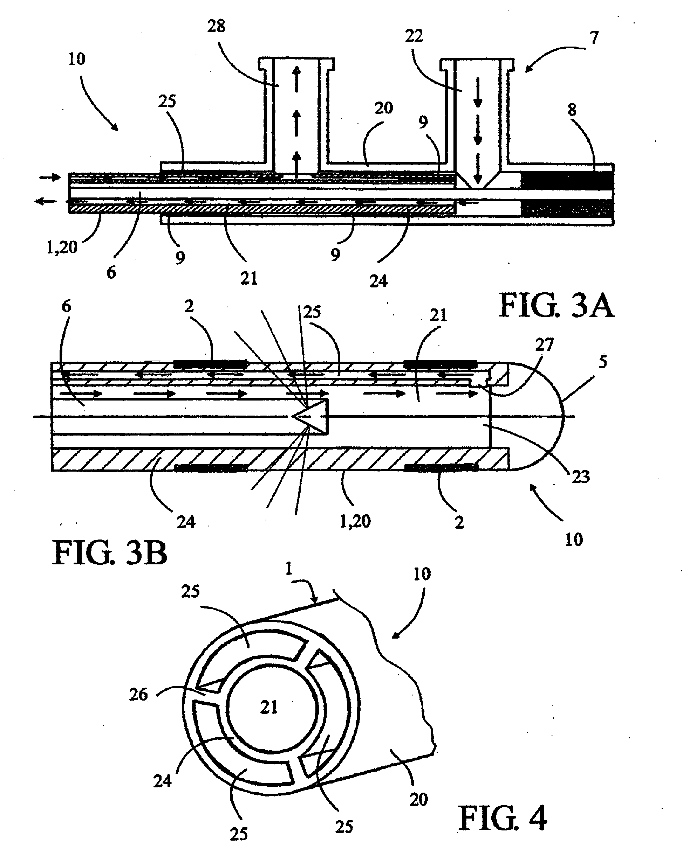

En référence à la figure 1, l'électrode intracérébrale 10 selon l'invention comporte un corps 1 étroit et allongé destiné à être implanté dans le cerveau d'un patient, ce corps étant pourvu de plots de contact 2 reliés électriquement à un appareil de mesure tel qu'un électroencéphalogramme (non représenté) par un connecteur 3 multi-contacts. Elle comporte un organe de montage 4 à vis ou similaire, agencé pour fixer le corps 1 de l'électrode 10 au crâne du patient. Le corps 1 de l'électrode est creux pour recevoir un instrument de traitement (non représenté) et pourvu d'une extrémité distale 5 fermée. Selon la nature de l'intervention, l'instrument de traitement introduit dans l'électrode intracérébrale 10 peut être un générateur de chaleur tel qu'une fibre optique 6 (cf. fig.3A et 3B) générant un faisceau laser, un générateur de froid par cryogénisation, ou similaire pour provoquer par exemple une thermolésion de la zone cérébrale ciblée. Bien entendu, ces exemples ne sont pas limitatifs.

En référence plus particulièrement aux figures 3A, 3B et 4, cette électrode intracérébrale 10 est complétée par un circuit intérieur de circulation de fluide fermé ménagé à l'intérieur du corps 1 jusqu'à proximité de l'extrémité distale fermée, et des moyens de connexion 7 extérieurs audit corps 1 pour connecter le circuit intérieur de fluide fermé à un circuit extérieur de circulation de fluide (non représenté) de sorte que le fluide, amené à travers le circuit extérieur de circulation de fluide, circule à l'intérieur de l'électrode 10 à travers le circuit intérieur de circulation de fluide.

Le fluide utilisé peut être un liquide physiologique ou tout autre fluide adéquat apte à transporter de la chaleur ou du froid, dans le but de réguler thermiquement l'électrode en fonction d'une consigne de température prédéterminée, contrôlée par le circuit extérieur de circulation de fluide et pilotée par une pompe pouvant faire varier le débit de fluide ou par tout système équivalent.

Le circuit intérieur de circulation de fluide comporte un tube 20 creux délimitant un canal central 21 pourvu au moins d'un orifice d'entrée 22 proximal et d'un orifice de sortie 23 distal à proximité de l'extrémité distale 5 de l'électrode 10. La paroi de ce tube comporte une paroi double 24 délimitant au moins un canal périphérique 25, et dans l'exemple trois canaux répartis sur la périphérie et séparés entre eux par une cloison radiale 26, ces canaux pouvant être de même section ou non. Au moins un de ces canaux 25 comporte au moins un orifice d'entrée 27 distal à proximité de l'extrémité distale 5 de l'électrode 10 et communiquant avec l'orifice de sortie 23 du canal central 21, et un orifice de sortie 28 proximal. Dans l'exemple représenté, l'orifice d'entrée 22 du canal central 21 et l'orifice de sortie 28 du canal périphérique 25 comportent des embouts et forment les moyens de connexion 7 à un circuit extérieur de circulation de fluide (non représenté), de sorte que le trajet aller du fluide s'effectue dans le canal central 21 et son trajet retour dans le ou les canaux périphériques 25. La construction inverse est également possible, c'est à dire que les moyens de connexion 7 comportent l'orifice d'entrée du canal périphérique 25 et l'orifice de sortie du canal central 21, de sorte que le trajet aller du fluide s'effectue dans le ou les canaux périphériques 25 et son trajet retour dans le canal central 21. Les moyens de connexion 7 forment un connecteur en une ou deux pièces assemblées entre elles par emboitement et solidarisées au tube 20 par collage dans les zones 9 pour créer l'étanchéité nécessaire et obturer les canaux périphériques 25 permettant de les séparer hydrauliquement du canal central 21 (cf. fig. 3A), ou par tout moyen équivalent.

Un des canaux périphériques 25 prévus dans le tube 20 délimite avantageusement un passage étanche et isolé pour les fils d'alimentation électrique (non représentés) des plots de contact 3, ces fils pouvant être surmoulés d'une matière synthétique électriquement isolante. Dans une variante de réalisation, les fils électriques peuvent être réalisés par un dépôt physique de métal sous vide (PVD) ou tout procédé équivalent.

Dans la réalisation de la figure 1, le tube 20 forme le corps 1 de l'électrode 10, alors que dans la variante de la figure 2, le tube 20 forme une pièce distincte 30 logée dans le corps 1 creux de l'électrode 11. Le corps 1 et/ou le tube 20 est fermé de manière étanche par un bouchon 8 à vis ou similaire. Ce bouchon 8 peut être également agencé pour fermer le corps 1 et/ou le tube 20 après avoir fait le vide d'air. Ce bouchon peut être inerte ou constitué d'un plot de contact. Il peut également être constitué d'un prisme de lecture pour imagerie cellulaire in vivo par microscopie confocale.

Dans tous les cas, le corps 1 de l'électrode 10 intégrant le tube 20, ou le corps 1 de l'électrode 11 et son tube 20 associé, peuvent être réalisés dans des matériaux perméables aux rayonnements magnétiques, tels que le polyamide, le polyéther bloc amide, le polycarbonate, le polytétrafluoroéthylène (PTFE), ou similaire, de sorte à pouvoir effectuer le traitement sous le contrôle d'un système d'imagerie par résonance magnétique (IRM).

L'électrode 10, 11 peut comporter au moins une fenêtre latérale (non représentée) à proximité de son extrémité distale 5, cette fenêtre étant transparente au rayonnement laser. Ce rayonnement laser est émis par une fibre optique 6 introduite dans le canal central 21 du tube 20 et dont l'extrémité peut comporter un cône inversé dont l'angle définit la forme du faisceau et donc la forme de la thermolésion. Le corps 1 de l'électrode 10, 11 peut être entièrement réalisé dans une matière synthétique transparente au rayonnement laser telle que le polyamide, le polyéther bloc amide, le polycarbonate, le polyimide, le polytétrafluoroéthylène ou similaires et résistante à une température d'au moins 150°C. Ce mode de réalisation permet de s'affranchir de la fenêtre latérale et de disposer ainsi de plus de liberté pour traiter par rayonnement laser sur toute la longueur de l'électrode 10, 11. Les plots de contact 2 sont formés par des pastilles ou des bagues et peuvent être collés ou surmoulés sur le corps 1 de l'électrode 10, 11. Dans une variante de réalisation, les plots de contact 2 peuvent être réalisés par un dépôt physique de métal sous vide (PVD) ou tout procédé équivalent. Ces plots de contact 2 sont distants d'un pas régulier par exemple égal à 5mm, ou d'un pas irrégulier. Ils doivent être réalisés dans des matériaux perméables aux rayonnements magnétiques, tels que le titane, les alliages de titane et de nickel, le carbone graphite, les alliages à base de cobalt, le tantale, les alliages de platine et d'iridium, les alliages non ferreux à base de cuivre, de nickel et de zinc, le zamac et les alliages de cuivre et de béryllium, ou similaire.

### Possibilités d'application industrielle:

La difficulté de conception des électrodes intracérébrales 10 et 11 telles que décrites réside dans leur faible diamètre et dans l'agencement du circuit de fluide dans un encombrement très restreint. A titre d'illustration uniquement, le diamètre extérieur des électrodes 10, 11 est par exemple compris entre 1.6 et 1.7 mm, le diamètre intérieur du canal central 21 est compris entre 0.8 et 0.9 mm, ce canal étant apte à recevoir une fibre optique 6 ou tout autre instrument de traitement dont le diamètre est compris entre 0.6 et 0.75 mm. Ces données chiffrées n'ont qu'une fonction d'illustration et ne sont en aucun cas des données limitatives.

Ces électrodes intracérébrales 10, 11 permettent au neurochirurgien d'effectuer une intervention en toute sécurité pour le patient et en limitant les interventions. Une ou plusieurs électrodes intracérébrales 10, 11 sont placées sur le crâne d'un patient et connectées à un électroencéphalogramme (EEG) pour contrôler l'activité cérébrale de certaines zones et détecter le cas échéant la ou les zones épileptogènes. Dès qu'une zone est identifiée, le neurochirurgien peut utiliser la ou les électrodes en place pour effectuer son traitement. Il introduit une fibre optique 6 dans le canal central 21 de l'électrode 10, 11 jusqu'à atteindre l'extrémité distale 5. Lorsque l'extrémité de la fibre optique 6 est en butée, il la retire de quelques millimètres pour se trouver face à la fenêtre latérale 9 si elle existe, ou entre deux plots de contact 3. Avant d'effectue la thermolésion, il connecte les orifices d'entrée 22 et de sortie 28 du circuit intérieur de circulation de fluide au circuit extérieur de circulation de fluide pour réguler la température de l'électrode 10, 11. Le fluide circule alors en boucle fermée en descendant vers l'extrémité distale de l'électrode 10, 11 par le canal central 21 autour de la fibre optique 6 puis en remontant par les canaux périphériques 25, ou inversement. Pendant ce temps, le neurochirurgien effectue la thermolésion en commandant l'alimentation électrique de la fibre optique 6. La thermolésion ne prendra que quelques secondes voire quelques minutes en fonction de la profondeur de la lésion. Après traitement, il peut faire un contrôle de la zone traitée à l'aide de l'électroencéphalogramme lui permettant de vérifier si la zone a disparu. Bien entendu, toute l'intervention peut être surveillée par un système d'imagerie médicale par résonance magnétique (IRM).

Il ressort clairement de cette description que l'invention permet d'atteindre les buts fixés. La présente invention n'est pas limitée aux exemples de réalisation décrits mais s'étend à toute modification et variante évidentes pour un homme du métier tout en restant dans l'étendue de la protection définie dans les revendications annexées.

## Revendications

1. Electrode intracérébrale (10, 11) comprenant un corps (1) étroit et allongé destiné à être implanté dans le cerveau d'un patient, ce corps étant pourvu de plots de contact (2) reliés électriquement à un appareil de mesure, et un organe de montage (4) agencé pour fixer ledit corps au crâne du patient, le corps de l'électrode étant creux agencé pour recevoir un instrument de traitement (6) et pourvu d'une extrémité distale (5) fermée, ladite électrode comportant un circuit intérieur (20) de circulation de fluide fermé ménagé à l'intérieur dudit corps (1) de l'électrode jusqu'à proximité de ladite extrémité distale (5) fermée, **caractérisée en ce que** ledit circuit intérieur de circulation de fluide comporte un tube creux (20) délimitant un canal central (21) pourvu au moins d'un orifice d'entrée (22) et d'un orifice de sortie (23), l'instrument de traitement (6) étant introduit dans ledit canal central (21) lors de l'utilisation de l'électrode, **en ce que** la paroi de ce tube creux (20) comporte une paroi double (24) délimitant au moins un canal périphérique (25) pourvu au moins d'un orifice d'entrée (27) et d'un orifice de sortie (28), **en ce que** ledit canal central (21) et ledit canal périphérique (25) communiquent entre eux par l'orifice de sortie (23) d'un des canaux (21) et l'orifice d'entrée (27) de l'autre canal (25) et **en ce que** ladite électrode comporte des moyens de connexion (7) extérieurs audit corps, comportant l'orifice d'entrée (22) d'un des canaux (21) et l'orifice de sortie (28) de l'autre canal (25), pour connecter ledit circuit intérieur de fluide fermé à un circuit extérieur de circulation de fluide de sorte que, lors de l'utilisation de l'électrode, ledit fluide, amené à travers ledit circuit extérieur, circule à l'intérieur de ladite électrode à travers ledit circuit intérieur de manière à contrôler et maîtriser la dissipation de chaleur ou de froid par ladite électrode et d'empêcher tout dégât collatéral dû à un traitement à l'aide dudit instrument de traitement.

2. Electrode selon la revendication 1, **caractérisée en ce que** ledit tube (20) forme le corps (1) de ladite électrode (10).

3. Electrode selon la revendication 1, **caractérisée en ce que** ledit tube (20) forme une pièce distincte (30) logée dans le corps (1) creux de ladite électrode (11).

4. Electrode selon la revendication 1, **caractérisée en ce que** ledit tube (20) comporte au moins deux canaux périphériques (25), un des canaux faisant partie du circuit intérieur de circulation du fluide, l'autre canal délimitant un passage étanche et isolé pour les fils d'alimentation électrique des plots de contact (2).

5. Electrode selon la revendication 1, **caractérisée en ce que** ledit corps (1) de l'électrode et/ou ledit tube (20) sont réalisés dans des matériaux perméables aux rayonnements magnétiques.

6. Electrode selon la revendication 1, **caractérisée en ce que** ledit corps (1) de l'électrode et/ou ledit tube (20) sont partiellement ou totalement transparents aux rayonnements laser.

7. Electrode selon l'une quelconque des revendications 5 et 6, **caractérisée en ce que** lesdits matériaux sont choisis dans le groupe comprenant le polyamide, le polyéther bloc amide, le polycarbonate, le polyimide, le polytétrafluoroéthylène.

8. Electrode selon la revendication 1, **caractérisée en ce qu'**elle comporte un bouchon (8) agencé pour fermer ledit corps (1) et/ou ledit tube (20) de manière étanche.

9. Electrode selon la revendication 8, **caractérisée en ce que** ledit bouchon (8) est agencé pour fermer ledit corps (1) et/ou ledit tube (20) sous vide d'air.

10. Electrode selon la revendication 1, **caractérisée en ce qu'**elle comporte au moins une fenêtre latérale à proximité de son extrémité distale (5) transparente aux rayonnements laser.

11. Electrode selon la revendication 1, **caractérisée en ce que** lesdits plots de contact (2) sont collés ou surmoulés sur ledit corps (1) de l'électrode (10, 11).

12. Electrode selon la revendication 1, **caractérisée en ce que** les plots de contact (2) et/ou les fils d'alimentation électrique sont réalisés par dépôt physique de métal sous vide.

13. Electrode selon l'une quelconque des revendications 11 et 12, **caractérisée en ce que** lesdits plots de contact (2) sont réalisés dans des matériaux perméables aux rayonnements magnétiques.

14. Electrode selon la revendication 13, **caractérisée en ce que** lesdits matériaux sont choisis dans le groupe comprenant le titane, les alliages de titane et de nickel, le carbone graphite, les alliages à base de cobalt, le tantale, les alliages de platine et d'iridium, les alliages non ferreux à base de cuivre, de nickel et de zinc, le zamac et les alliages de cuivre et de béryllium.

## Patentansprüche

1. Gehirnelektrode (10, 11) bestehend aus einem schmalen und länglichem Körper (1), der dazu bestimmt ist, im Gehirn eines Patienten implantiert zu werden, wobei dieser Körper mit elektrisch mit einem Messgerät verbundenen Kontaktpunkten (2) versehen ist, und einer Montagevorrichtung (4), die ausgelegt ist, um den besagten Körper am Schädel des Patienten zu befestigen, wobei der Körper der Elektrode hohl ist und ausgelegt ist, um ein Behandlungs-Instrument (6) aufzunehmen und mit einem geschlossenen distalen Ende (5) versehen ist, wobei die besagte Elektrode einen innerhalb des besagten Körpers (1) der Elektrode bis in die Nähe des geschlossenen distalen Endes (5) angeordneten internen geschlossenen Kreislauf (20) für die Zirkulation von Fluid beträgt, **dadurch gekennzeichnet, dass** der besagte interne Kreislauf für die Zirkulation von Fluid in hohles Rohr (20) aufweist, das einen zentralen Kanal (21) begrenzt, der zumindest mit einer Einlass-Öffnung (22) und einer Auslass-Öffnung (23) versehen ist, wobei das Behandlungs-Instrument (6) bei der Verwendung der Elektrode in den besagten zentralen Kanal (21) eingeführt wird, dadurch, dass die Wand dieses hohlen Rohrs (20) eine Doppelwand (24) beträgt, die zumindest einen Umfangs-Kanal (25) begrenzt, der zumindest mit einer Einlass-Öffnung (27) und einer Auslass-Öffnung (28) versehen ist, dadurch, dass der besagte zentrale Kanal (21) und der besagte Umfangs-Kanal (25) über die Auslass-Öffnung (23) eines der Kanäle (21) und die Einlass-Öffnung (27) des anderen Kanals (25) miteinander in Verbindung stehen, und dadurch, dass die besagte Elektrode außerhalb des besagten Körpers befindliche Anschlussmittel (7) beträgt, die die Einlass-Öffnung (22) eines der Kanäle (21) und die Auslass-Öffnung (28) des anderen Kanals (25) tragen, um den besagten internen geschlossenen Fluid-Kreislauf mit einem externen Fluid-Zirkulations-Kreislauf zu verbinden so dass, bei der Verwendung der Elektrode, das besagte über den besagten externen Kreislauf zugeführte Fluid in der besagten Elektrode durch den besagten internen Kreislauf fließt, um die Wärme- oder Kälte-Ableitung durch die besagte Elektrode zu kontrollieren und zu steuern und jegliche auf die Behandlung durch das besagte Behandlungs-Instrument zurückzuführende Folgeschäden zu vermeiden.

2. Elektrode nach Anspruch 1, **dadurch gekennzeichnet, dass** das besagte Rohr (20) den Körper (1) der besagten Elektrode (10) bildet.

3. Elektrode nach Anspruch 1, **dadurch gekennzeichnet, dass** das besagte Rohr (20) ein separates Teil (30) bildet, das in den hohlen Körper (1) der besagten Elektrode (11) eingesetzt wird.

4. Elektrode nach Anspruch 1, **dadurch gekennzeichnet, dass** das besagte Rohr (20) zumindest zwei Umfangs-Kanäle (25) beträgt, wobei einer der Kanäle Bestandteil des internen Fluid-Zirkulations-Kreislaufs ist und der andere Kanal einen dichten und isolierten Durchgang für die Stromversorgungsdrähte der Kontaktpunkte (2) begrenzt.

5. Elektrode nach Anspruch 1, **dadurch gekennzeichnet, dass** der besagte Körper (1) der Elektrode und/oder das besagte Rohr (20) aus für magnetische Strahlungen durchlässige Materialien hergestellt sind.

6. Elektrode nach Anspruch 1, **dadurch gekennzeichnet, dass** der besagte Körper (1) der Elektrode und/oder das besagte Rohr (20) zum Teil oder in Gänze für Laserstrahlung durchlässig sind.

7. Elektrode nach einem beliebigen der Ansprüche 5 und 6, **dadurch gekennzeichnet, dass** die besagten Materialien in der Gruppe bestehend aus Polyamid, Polyetherblockamid, Polykarbonat, Polyimid, Polytetrafluorethylen gewählt werden.

8. Elektrode nach Anspruch 1, **dadurch gekennzeichnet, dass** sie einen Verschluss (8) beträgt, der angeordnet ist, um den besagten Körper (1) und/oder das besagte Rohr (20) dicht zu verschließen.

9. Elektrode nach Anspruch 8, **dadurch gekennzeichnet, dass** der besagte Verschluss (8) ausgelegt ist, um den besagten Körper (1) und/oder das besagte Rohr (20) unter Vakuum zu verschließen.

10. Elektrode nach Anspruch 1, **dadurch gekennzeichnet, dass** sie zumindest ein für Laserstrahlung durchlässiges Seitenfenster in der Nähe ihres distalen Endes (5) beträgt.

11. Elektrode nach Anspruch 1, **dadurch gekennzeichnet, dass** die besagten Kontaktpunkte (2) auf den besagten Körper (1) von Elektrode (10, 11) aufgeklebt oder vergossen sind.

12. Elektrode nach Anspruch 1, **dadurch gekennzeichnet, dass** die Kontaktpunkte (2) und/oder die Stromversorgungsdrähte durch physikalische Metallablagerung unter Vakuum hergestellt sind.

13. Elektrode nach einem beliebigen der Ansprüche 11 und 12, **dadurch gekennzeichnet, dass** die besagten Kontaktpunkte (2) aus für magnetische Strahlungen durchlässige Materialien hergestellt sind.

14. Elektrode nach Anspruch 13, **dadurch gekennzeichnet, dass** die besagten Materialien in der Gruppe bestehend aus Titan, Titan- und Nickel-Legierungen, Graphitkohlenstoff, Legierungen auf Kobalt-Basis, Tantal, Platin- und Iridium-Legierungen, nichteisenhaltige Legierungen auf Kupfer-, Nickel- und Zink-Basis, Zamak und Kupfer- und Beryllium-Legierungen gewählt werden.

## Claims

1. Intracerebral electrode (10, 11) including a narrow, elongated body (1) that is intended to be implanted in the brain of a patient, this body being provided with contact pads (2) that are electrically connected to a measuring apparatus, and a mounting member (4) that is set up so as to attach said body to the head of the patient, the electrode body being hollow in order to receive a treatment instrument (6) and being provided with a closed distal end (5), said electrode comprises a closed inner fluid flow circuit (20) provided inside said electrode body (1) up to an area near said closed distal end (5), **characterized in that** said closed inner fluid flow circuit comprises a hollow tube (20) that delimits a central channel (21) provided with at least one inlet opening (22) and one outlet opening (23), the treatment instrument (6) being introduced in said central channel (21) when the electrode is used, **in that** the wall of this hollow tube (20) comprises a double wall (24) that delimits at least one peripheral channel (25) provided with at least one inlet opening (27) and one outlet opening (28), **in that** said central channel (21) and said peripheral channel (25) communicate with each other through the outlet opening (23) of one of the channels (21) and the inlet opening (27) of the other channel (25) and **in that** said electrode comprises connection means (7) located outside said body, including the inlet opening (22) of one of the channels (21) and the outlet opening (28) of the other channel (25) so as to connect said closed inner fluid flow circuit to an outer fluid flow circuit so that said fluid, moved through said outer fluid flow circuit, flows into said electrode through said inner circuit in order to control and master the dissipation of heat or cold by said electrode and to prevent any collateral damage due to a treatment with the help of said treatment instrument.

2. Electrode according to claim 1, **characterized in that** said tube (20) forms the body (1) of said electrode (10).

3. Electrode according to claim 1, **characterized in that** said tube (20) forms a separate part (30) accommodated in the hollow body (1) of said electrode (11).

4. Electrode according to claim 1, **characterized in that** said tube (20) comprises at least two peripheral channels (25), one of the channels belonging to the inner fluid flow circuit while the other channel delimits a sealed and insulated passage for the electrical power supply wires of the contact pads (2).

5. Electrode according to claim 1, **characterized in that** said electrode body (1) and/or said tube (20) are made out of materials permeable to magnetic radiation.

6. Electrode according to claim 1, **characterized in that** said electrode body (1) and/or said tube (20) are partly or totally transparent to laser radiation.

7. Electrode according to any of claims 5 and 6, **characterized in that** said materials are chosen in the group comprising polyamide, polyether block amide, polycarbonate, polyimide, polytetrafluoroethylene.

8. Electrode according to claim 1, **characterized in that** it comprises a plug (8) arranged to close said body (1) and/or said tube (20) in a tight manner.

9. Electrode according to claim 8, **characterized in that** said plug (8) is arranged to close said body (1) and/or said tube (20) under vacuum.

10. Electrode according to claim 1, **characterized in that** it comprises at least one side window, transparent to laser radiation, near its distal end (5).

11. Electrode according to claim 1, **characterized in that** said contact pads (2) are glued or overmolded on said body (1) of the electrode (10, 11).

12. Electrode according to claim 1, **characterized in that** the contact pads (2) and/or the electrical power supply wires are formed by physical vacuum deposition of metal.

13. Electrode according to any of claims 11 and 12, **characterized in that** said contact pads (2) are made out of materials permeable to magnetic radiation.

14. Electrode according to claim 13, **characterized in that** said materials are chosen in the group comprising titanium, titanium and nickel alloys, carbon graphite, cobalt-based alloys, tantalum, platinum and iridium alloys, copper, nickel and zinc-based nonferrous alloys, zamak, copper and beryllium alloys.
